(19) Europäisches Patentamt
European Patent Office
Office européen des brevets

(11) EP 4 635 943 A2

(12) **EUROPEAN PATENT APPLICATION**

(43) Date of publication:
**22.10.2025 Bulletin 2025/43**

(21) Application number: **25194216.5**

(22) Date of filing: **17.02.2020**

(51) International Patent Classification (IPC):
***C07D 221/18*** *(2006.01)*

(52) Cooperative Patent Classification (CPC):
**C07D 221/18**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2019 GB 201915911**

(62) Document number(s) of the earlier application(s) in accordance with Art. 76 EPC:
**20707793.4 / 4 051 631**

(71) Applicant: **Macfarlan Smith Limited**
**Edinburgh EH11 2QA (GB)**

(72) Inventor: **The designation of the inventor has not yet been filed**

(74) Representative: **Marks & Clerk LLP**
**15 Fetter Lane**
**London EC4A 1BW (GB)**

Remarks:
•This application was filed on 05-08-2025 as a divisional application to the application mentioned under INID code 62.
•Claims filed after the date of filing of the application (Rule 68(4) EPC).

# (54) PRODUCTION PROCESS OF A HYDROCHLORIC ACID SALT OF APOMORPHINE AND DERIVATIVES THEREOF

(57) The present invention provides processes for the preparation of a solid hydrochloric acid salt of a compound of formula (2):

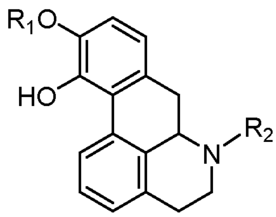

Compound of formula (2)

wherein:
$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and
$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1-20}$-alkyl-$C_{3-20}$-cycloalkyl, substituted -$C_{1-20}$-alkyl-$C_{3-20}$-cycloalkyl, unsubstituted allyl and substituted allyl.

EP 4 635 943 A2

## Description

The present invention provides processes for the preparation of apomorphine and apocodeine. In particular, the invention provides improved processes for the preparation of apomorphine and apocodeine.

Apomorphine has the IUPAC chemical name of (6a*R*)-5,6,6a,7-tetrahydro-6-methyl-4*H*-dibenzo[de,g]quinoline-10,11-diol. It is a non-narcotic morphine derivative which can be used as an emetic, as well as in the treatment of Parkinson's disease.

Apomorphine may be prepared from morphine by an acid catalysed dehydration and alkyl migration reaction (see Small et al, J. Org. Chem., 1940, 5, 334):

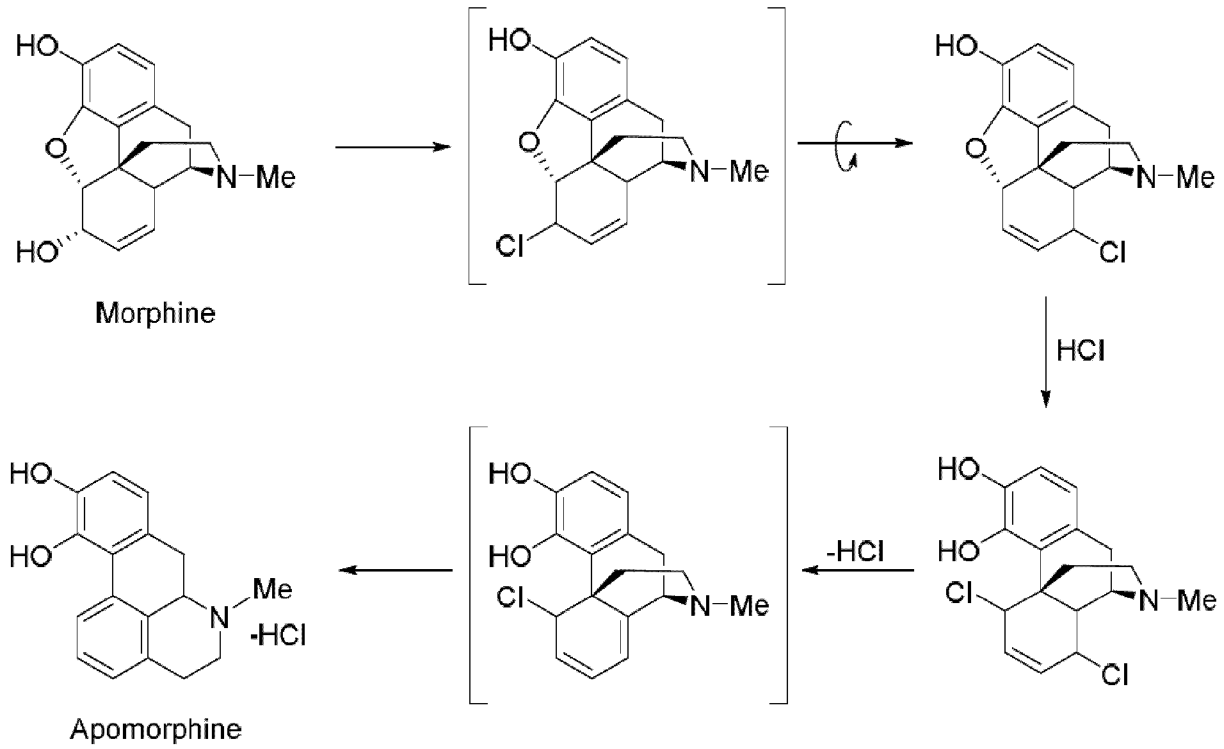


Apomorphine can be prepared by adding a heated solution of morphine in hydrochloric acid to a concentrated solution of calcium chloride in aqueous hydrochloric acid. The calcium chloride hydrochloric acid melt is heated before addition of the acidic solution of morphine. On heating the solution, copious anhydrous hydrogen chloride gas is liberated, which corrodes vent lines and requires abatement through scrubbers.

Furthermore, the apomorphine reaction is carried out at temperatures in excess of 100 °C and the conditions are so aggressive that high levels of an impurity, the morphine-apomorphine dimer, are generated. The dimer is difficult to remove from the formed apomorphine hydrochloride. It is identified in the European Pharmacopeia for Apomorphine Hydrochloride as Impurity C:

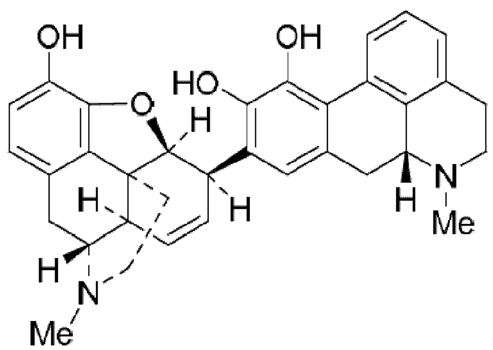

Impurity C (morphine-apomorphine dimer) (6a,*R*)-9-[7,8-didehydro-4,5α-epoxy-3-hydroxy-17-methylmorphinan-6α-yl]-6-methyl-5,6,6a,7-tetrahydro-4*H*-dibenzo[*de*,*g*]quinoline-10,11-diol

Apocodeine has the IUPAC chemical name (6a*R*)-5,6,6a,7-tetrahydro-10-methoxy-6-methyl-4H-dibenzo[de,g] quinoline-10,11-diol. It may be prepared by reacting codeine with glacial phosphoric acid at 175° (see Small et al, J. Org. Chem., 1940, 5, 334). Apocodeine used therapeutically as an emetic.

We have developed improved processes which overcomes the disadvantages associated with prior art methods. The present processes are suitable for the large-scale or industrial manufacture of apomorphine or apocodeine.

Definitions

The point of attachment of a moiety or substituent is represented by "-". For example, -OH is attached through the oxygen atom.

"Alkyl" refers to a straight-chain or branched saturated hydrocarbon group. In certain embodiments, the alkyl group may have from 1-20 carbon atoms, in certain embodiments from 1-15 carbon atoms, in certain embodiments, 1-8 carbon atoms. The alkyl group may be unsubstituted. Alternatively, the alkyl group may be substituted. Unless otherwise specified, the alkyl group may be attached at any suitable carbon atom and, if substituted, may be substituted at any suitable atom. Typical alkyl groups include but are not limited to methyl, ethyl, n-propyl, iso-propyl, n-butyl, iso-butyl, sec-butyl, tert-butyl, n-pentyl, n-hexyl, and the like.

The term "cycloalkyl" is used to denote a saturated carbocyclic hydrocarbon radical. The cycloalkyl group may have a single ring or multiple condensed rings. In certain embodiments, the cycloalkyl group may have from 3-15 carbon atoms, in certain embodiments, from 3-10 carbon atoms, in certain embodiments, from 3-8 carbon atoms. The cycloalkyl group may be unsubstituted. Alternatively, the cycloalkyl group may be substituted. Unless other specified, the cycloalkyl group may be attached at any suitable carbon atom and, if substituted, may be substituted at any suitable atom. Typical cycloalkyl groups include but are not limited to cyclopropyl, cyclobutyl, cyclopentyl, cyclohexyl and the like.

The term "alcohol solvent" refers to a liquid alcohol in which the hydrochloric acid salt of the compound of formula (2) is insoluble or substantially insoluble but in which Impurity C and/or other impurities are more soluble than the hydrochloric acid salt of the compound of formula (2).

The term "ambient temperature" means one or more room temperatures between about 15 °C to about 30 °C, such as about 15 °C to about 25 °C.

The term "consisting" is closed and excludes additional, unrecited elements or method steps in the claimed invention.

The term "consisting essentially of" is semi-closed and occupies a middle ground between "consisting" and "comprising". "Consisting essentially of" does not exclude additional, unrecited elements or method steps which do not materially affect the essential characteristic(s) of the claimed invention.

The term "comprising" is inclusive or open-ended and does not exclude additional, unrecited elements or method steps in the claimed invention. The term is synonymous with "including but not limited to". The term "comprising" encompasses three alternatives, namely (i) "comprising", (ii) "consisting", and (iii) "consisting essentially of".

The term "impurity" refers to a compound which is undesirably present and typically occurs in small quantities. The impurity may be present in the starting material, produced during the course of the reaction and/or is present in the product. Impurity C described above is an impurity named in the European Pharmacopeia for Apomorphine Hydrochloride.

"Halo" or "halogen" refers to -F, -Cl, -Br and -I e.g. -Cl, -Br and -I.

The term "overnight" refers to the period of time between the end of one working day to the subsequent working day in which a time frame of about 12 to about 18 hours has elapsed between the end of one procedural step and the instigation of the following step in a procedure.

"Slurry" means a heterogeneous mixture of at least a portion of the solid hydrochloric acid salt of the compound of formula (2) in a solvent mixture comprising an alcohol solvent and hydrochloric acid. "Slurry" therefore includes a mixture of the hydrochloric acid salt of the compound of formula (2) which is partially present as a solid, as well as being partially dissolved in the solvent mixture.

"Substituted" refers to a group in which one or more (e.g. 1, 2, 3, 4 or 5) hydrogen atoms are each independently replaced with substituents which may be the same or different. The substituent may be any group which tolerates the alkylation reaction conditions. Examples of substituents include but are not limited to -halo,-$CF_3$, -$R_a$, -O-$R_a$, and -$NR_aR_b$, , such as -halo,-$CF_3$, -$R_a$, and -$NR_aR_b$. $R_a$ and $R_b$ are independently selected from the groups consisting of H, alkyl, and cycloalkyl, and wherein $R_a$ and $R_b$ may be unsubstituted or further substituted as defined herein.

Detailed Description

In one aspect, the present invention provides a process for the preparation of a hydrochloric acid salt of

compound of formula (2), the process comprising the steps of:

(a) heating calcium chloride and water to a temperature greater than 100 °C to form an aqueous calcium chloride solution;
(b) adding an aqueous hydrochloric acid solution of a compound of formula (1) to the aqueous calcium chloride solution; and
(c) heating the reaction mixture of step (b) to a temperature greater than 100 °C to form the hydrochloric acid salt of the compound of formula (2);

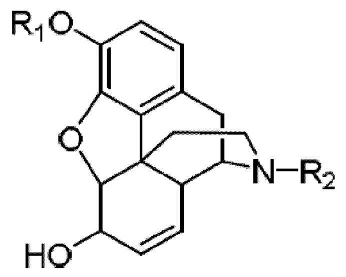

Compound of formula (1)

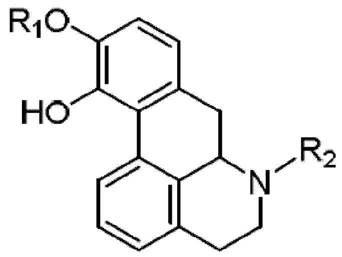

Compound of formula (2)

wherein:

$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and
$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1-20}$-alkyl-$C_{3-20}$-cycloalkyl, substituted -$C_{1-20}$-alkyl-$C_{3-20}$-cycloalkyl, unsubstituted allyl and substituted allyl.

In step (a), calcium chloride and water are heated to a temperature greater than 100 °C to form a calcium chloride solution. The calcium chloride inputted into step (a) may be anhydrous or a hydrate. In one embodiment, the calcium chloride is anhydrous. In another embodiment, the calcium chloride is a hydrate, for example, calcium chloride dihydrate.

The w/w (weight/weight) ratio of calcium chloride to water can be any suitable w/w ratio provided a solution is formed on heating. The quantity of water present in the calcium chloride (i.e. the water of hydration) may be taken into account when calculating the total quantity of water to be used. The w/w ratio of calcium chloride to water may be in the range from about 1g calcium chloride : about 0.01 to about 1 g of water. In one embodiment, the w/w ratio is about 1g calcium chloride : about 0.6 g of water.

The calcium chloride and water may be heated to one or more temperatures in the range of ≥ about 100 °C to about ≤ about 200 °C. In some embodiments, the calcium chloride and water may be heated to one or more temperatures ≥ about 110 °C. In some embodiments, the calcium chloride and water may be heated to one or more temperatures ≥ about 120 °C. In some embodiments, the calcium chloride and water may be heated to one or more temperatures ≥ about 130 °C. In some embodiments, the calcium chloride and water may be heated to one or more temperatures ≥ about 140 °C. In some embodiments, the calcium chloride and water may be heated to one or more temperatures ≤ about 190 °C. In some embodiments, the calcium chloride and water may be heated to one or more temperatures ≤ about 180 °C. In some embodiments, the calcium chloride and water may be heated to one or more temperatures ≤ about 170 °C. In some embodiments, the calcium chloride and water may be heated to one or more temperatures ≤ about 160 °C. In some embodiments, calcium chloride and water may be heated to one or more temperatures ≤ about 150 °C. In one embodiment, the calcium chloride and water may be heated to one or more temperatures in the range of about ≥ about 140 °C to about ≤ about 150 °C. In one embodiment, the calcium chloride and water may be heated to about 145 °C.

The initial calcium chloride/water mixture may be an immobile slurry or damp solid at room temperature. On heating, however, the calcium chloride will start to dissolve and agitation may be of assistance. The formation of the calcium chloride solution may be encouraged through the use of an aid such as stirring, and/or shaking.

The calcium chloride solution does not comprise additional hydrochloric acid. In one embodiment, the aqueous calcium chloride solution of step (a) consists essentially of calcium chloride and water. It has been found that the hydrochloric acid may be omitted from the solution without detriment to the impurity profile of the formed compound (2). Nor is the absence of the hydrochloric acid detrimental to the yield of the process when compared to the equivalent process

when hydrochloric acid is present. Furthermore, the absence of the acid from the solution means that the anhydrous hydrogen chloride gas is not liberated on forming the solution and, as such, vent lines remain uncorroded and abatement through scrubbers is not required in this step of the process.

In step (b), an aqueous hydrochloric acid solution of a compound of formula (1) is added to the heated calcium chloride solution.

$R_1$ may be selected from the group consisting of -H, an unsubstituted straight-chain $C_1$-$C_{20}$-alkyl, unsubstituted branched-chain $C_1$-$C_{20}$-alkyl, and unsubstituted cyclic $C_3$-$C_{20}$-alkyl. $R_1$ may be selected from the group consisting of -H and an unsubstituted straight-chain $C_1$-$C_{20}$-alkyl, such as - H or -Me. In one embodiment, $R_1$ may be -H. In another embodiment, $R_1$ may be -Me. In yet another embodiment, $R_1$ may be propyl, for example, n-propyl or i-propyl, such as n-propyl.

$R_2$ may be selected from the group consisting of an unsubstituted straight-chain $C_1$-$C_{20}$-alkyl, unsubstituted branched-chain $C_1$-$C_{20}$-alkyl, unsubstituted cyclic $C_3$-$C_{20}$-alkyl, unsubstituted -$C_{1-20}$-alkyl-$C_{3-20}$-cycloalkyl, and unsubstituted allyl. For example, $R_2$ may be a cyclopropylmethyl (i.e.

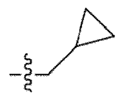

), cyclobutylmethyl (i.e.

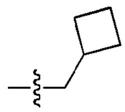

) or allyl group (i.e.

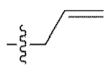

). In one embodiment, $R_2$ is a cyclopropylmethyl group. In another embodiment, $R_2$ may be a methyl group.

The aqueous hydrochloric acid solution may be prepared from water and concentrated hydrochloric acid. Any suitable v/v ratio of water : acid may be used. For example, the v/v ratio of water : acid may be from about 100 : 0.01 to about 0.01 : 100, such as about 100 : 1 to about 1 : 100. In one embodiment, the v/v ratio of water : acid is from about 1 : 10 to about 10 : 1, for example, about 1: about 3.1.

Alternatively, hydrogen chloride gas may be bubbled through water to form the aqueous hydrochloric acid solution.

The quantities of water and/or acid are not particularly limiting provided there is enough water and/or acid to substantially dissolve the compound (1) and the water and/or acid do not significantly adversely affect the reaction. The quantity of water present in the calcium chloride solution and/or compound (1) (which may also be used wet) may be taken into account when calculating the total quantity of water to be used. The quantity of water present in the acid may also be taken into account when calculating the total quantity of water to be used.

The compound (1) is substantially dissolved in the water and acid. The dissolution of the compound (1) may be encouraged through the use of an aid such as stirring, and/or shaking.

The compound of formula (1) may have chiral centres at C-5, C-6, C-9 and C-13. The compound (1) may have the stereochemistry shown below:

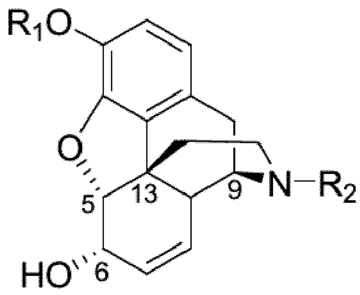

Compound of formula (1)

The compound of formula (2) may have a chiral centre at C-6, and may have the stereochemistry shown below:

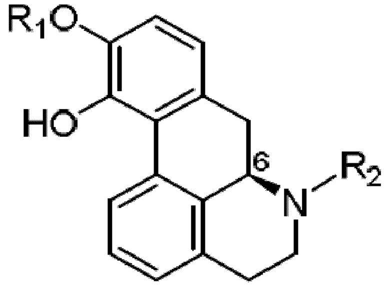

Compound of formula (2)

When $R_1$ is -H and $R_2$ is a methyl (-Me) group. In this instance, the compound of formula (1) is morphine, and the corresponding compound of formula (2) is apomorphine.

When $R_1$ and $R_2$ are methyl groups. In this instance, the compound of formula (1) is codeine, and the corresponding compound of formula (2) is apocodeine.

The aqueous hydrochloric acid solution of compound (1) may be added to the heated calcium chloride solution portionwise, such that frothing or foaming is minimised or substantially eliminated.

In step (c), the reaction mixture of step (b) is heated at a temperature greater than 100 °C to form the hydrochloric acid salt of the compound of formula (2).

The reaction mixture may be heated to one or more temperatures in the range of ≥ about 100 °C to about ≤ about 200 °C. In some embodiments, the reaction mixture may be heated to one or more temperatures ≥ about 110 °C. In some embodiments, the reaction mixture may be heated to one or more temperatures ≥ about 120 °C. In some embodiments, the reaction mixture may be heated to one or more temperatures ≥ about 130 °C. In some embodiments, the reaction mixture may be heated to one or more temperatures ≥ about 140 °C. In some embodiments, the reaction mixture may be heated to one or more temperatures ≤ about 190 °C. In some embodiments, the reaction mixture may be heated to one or more temperatures ≤ about 180 °C. In some embodiments, the reaction mixture may be heated to one or more temperatures ≤ about 170 °C. In some embodiments, the reaction mixture may be heated to one or more temperatures ≤ about 160 °C. In some embodiments, the reaction mixture may be heated to one or more temperatures ≤ about 150 °C. In one embodiment, the reaction mixture may be heated to one or more temperatures in the range of about ≥ about 140 °C to about ≤ about 150 °C. In one embodiment, the reaction mixture may be heated to a temperature in the range of about 143 °C to about 145 °C.

The temperature to which the reaction is heated is not particularly limiting provided it is suitably high enough such that a calcium chloride solution is produced and sufficient or substantially complete conversion of the compound (1) to compound (2) occurs, and suitably low enough such that unacceptably high levels of Impurity C are not generated.

The process may be conducted under an inert atmosphere (e.g. under nitrogen or argon gas).

The reaction is carried out for a period of time until it is determined that the reaction is complete or substantially complete. Completion of the reaction may be determined by in-process analysis. Typically, the reaction is complete within about 2 hours.

On completion of the reaction, hydrochloric acid (e.g. dilute hydrochloric acid) may be added to work-up the reaction mixture. The acid may be added portionwise over a period time (e.g. about 30 minutes). The temperature of the worked up reaction mixture may be maintained at one or temperatures as described above. The reaction vessel may be cooled to a temperature below those described above (e.g. about 55 °C).

The suspension formed, which comprises the hydrochloric acid salt of the compound of formula (2), may be recovered by filtering, decanting or centrifuging. Howsoever the product of the invention is recovered, the separated product may be washed with acid (e.g. dilute hydrochloric acid) and dried. Drying may be performed using known methods, for example, at temperatures in the range of about 10 °C to about 60 °C, such as about 20 °C to about 40 °C, for example, ambient temperature under vacuum (for example about 1 mbar to about 30 mbar) for about 1 hour to about 24 hours. It is preferred that the drying conditions are maintained below the point at which the hydrochloric acid salt of the compound of formula (2) degrades and so when the hydrochloric acid salt of the compound of formula (2) is known to degrade within the temperature or pressure ranges given above, the drying conditions should be maintained below the degradation temperature or vacuum.

The compound of formula (1) may be O-demethylated before it is reacted in the process of the invention. In this instance, $R_1$ is methyl in the compound (1) and is -H in the obtained compound (1).

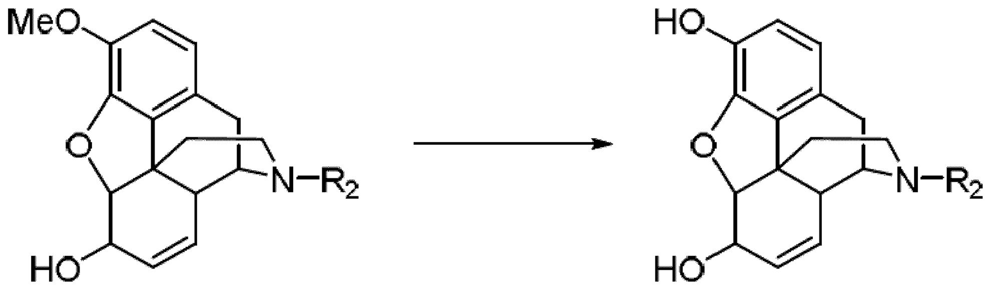

Compound of formula (1) $R_1$ = Me

Compound of formula (1) $R_1$ = H

Alternatively, the compound of formula (2) may be O-demethylated after it is reacted in the process of the invention. In this instance, $R_1$ is methyl in the compound (2) and is -H in the obtained compound (2).

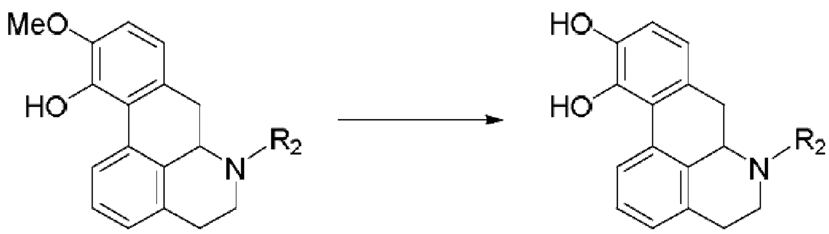

Compound of formula (2) $R_1$ = Me

Compound of formula (2) $R_1$ = H

O-demethylation reagents include but are not limited to boron tribromide, aluminium chloride, methionine in methanesulfonic acid, pyridine hydrochloride and mixtures thereof.

In another aspect, the present invention provides a process for preparing a solid hydrochloric acid salt of compound (2), the process comprising the step of treating the hydrochloric acid salt of compound (2) with a solvent mixture comprising an alcohol solvent and hydrochloric acid at a temperature greater than ambient temperature,

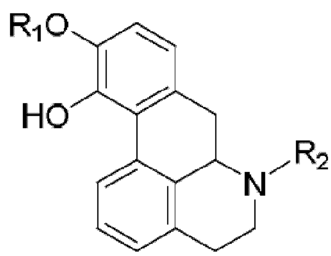

Compound of formula (2)

wherein:

$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and
$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1-20}$-alkyl-$C_{3-20}$-cycloalkyl, substituted -$C_{1-20}$-alkyl-$C_{3-20}$-cycloalkyl, unsubstituted allyl and substituted allyl.
$R_1$ and $R_2$ are as described above for the first aspect of the invention.

The inventors have found that treating the hydrochloric acid salt of compound (2) with a solvent mixture comprising an alcohol solvent and hydrochloric acid at a temperature greater than ambient temperature results in a product (i.e. the hydrochloric acid salt of compound (2)) with improved purity. Additionally, assays of the product demonstrate higher quantities of the compound (2). Furthermore, the appearance of product is improved, which means the product contains fewer coloured impurities. Without wishing to be bound by theory, it is believed that the impurities are more soluble in the alcohol solvent than the compound of formula (2). As such, the impurities can be dissolved in the alcohol solvent and selectively removed from the desired hydrochloric acid salt of compound (2).
The hydrochloric acid salt of compound (2) is as described above.
The hydrochloric acid salt of the compound (2) may be slurried in the solvent mixture. The solvent mixture comprises an alcohol solvent and hydrochloric acid. The alcohol solvent includes but are not limited to methanol, ethanol, propanol, butanol (n-, i- or t-), pentanols, cyclopentanol, hexanols, cyclohexanol, or a mixture thereof. In one embodiment, the alcohol solvent may comprise methanol, ethanol or a mixture thereof. In one embodiment, the alcohol solvent may be Alcohol M (i.e. 96% ethanol denatured with 4% methanol).
The hydrochloric acid present in the solvent mixture may be aqueous hydrochloric acid, such as concentrated hydrochloric acid (37% hydrochloric acid in water). In this instance, the process comprises water. The presence of water is not detrimental to impurity profile of the product.
Alternatively, hydrogen chloride gas may be bubbled through the alcohol solvent to form an acidic alcohol solvent.
The treatment of the hydrochloric acid salt of the compound (2) is carried out at one or more temperatures greater than ambient temperature i.e. greater than 30°C and below the boiling point of the reaction mixture. The boiling point of the reaction mixture may vary depending on the pressure under which the process is conducted. In one embodiment, the treating step is carried out at atmospheric pressure (i.e. $1.0135 \times 10^5$ Pa). In one embodiment, the process may be carried out at one or more temperatures in the range of ≥ about 40 °C to about ≤ about 70 °C. In some embodiments, the process is carried out at one or more temperatures ≥ about 45 °C. In some embodiments, the process is carried out at one or more temperatures ≥ about 50 °C. In some embodiments, the process is carried out at one or more temperatures ≥ about 55 °C. In some embodiments, the process is carried out at one or more temperatures ≥ about 60 °C. In some embodiments, the process is carried out at one or more temperatures ≤ about 69 °C. In some embodiments, the process is carried out at one or more temperatures ≤ about 68 °C. In some embodiments, the process is carried out at one or more temperatures ≤ about 67 °C. In some embodiments, the process is carried out at one or more temperatures ≤ about 66 °C. In some embodiments, the process is carried out at one or more temperatures ≤ about 65 °C. In one embodiment, the process is carried out at one or more temperatures in the range of ≥ about 55 °C to about ≤ 65 °C, such as about 60 °C.
The process is carried out for a period of time until it is determined that the purification is complete or substantially complete. Completion of the purification process may be determined by in-process analysis. Typically, the purification process is complete within about 2 hours.
The reaction mixture may be cooled to ambient temperature or a temperature of less than ambient temperature. In one embodiment, the reaction mixture may be cooled to one or more temperatures in the range of ≥ about 0 °C to about ≤ 20 °C. In some embodiments, the reaction mixture is cooled to one or more temperatures ≥ about 1 °C. In some embodiments, the reaction mixture is cooled to one or more temperatures ≥ about 2 °C. In some embodiments, the reaction mixture is cooled to one or more temperatures ≥ about 3 °C. In some embodiments, the reaction mixture is cooled to one or more temperatures ≥ about 4 °C. In some embodiments, the reaction mixture is cooled to one or more temperatures ≥ about 5 °C. In some embodiments, the reaction mixture is cooled to one or more temperatures ≤ about 15 °C. In some embodiments, the reaction mixture is cooled to one or more temperatures ≤ about 14 °C. In some embodiments, the reaction mixture is cooled to one or more temperatures ≤ about 13 °C. In some embodiments, the reaction mixture is cooled to one or more temperatures ≤ about 12 °C. In some embodiments, the reaction mixture may be cooled to one or more temperatures ≤ about 11 °C. In some embodiments, the reaction mixture is cooled to one or more temperatures ≤ about 10 °C. In one embodiment, the reaction mixture is cooled to one or more temperatures in the range of about 5°C to about 10 °C.
On completion of the purification process, the hydrochloric acid salt of the compound of formula (2) may be recovered by filtering, decanting or centrifuging. Howsoever the product of the invention is recovered, the separated

product may be washed with solvent (e.g.one or more of the alcohol solvents described above) and dried. Drying may be performed using known methods, for example, at temperatures in the range of about 10 °C to about 60 °C, such as about 20 °C to about 40 °C, for example, ambient temperature under vacuum (for example about 1 mbar to about 30 mbar) for about 1 hour to about 24 hours. It is preferred that the drying conditions are maintained below the point at which the hydrochloric acid salt of the compound of formula (2) degrades and so when the hydrochloric acid salt of the compound of formula (2) is known to degrade within the temperature or pressure ranges given above, the drying conditions should be maintained below the degradation temperature or vacuum.

In carrying out the process of the invention, it is possible to obtain a product (the hydrochloric acid salt of compound (2)) with an improved impurity profile. In one embodiment, it is possible to significantly reduce the levels of Impurity C in apomorphine hydrochloride, an impurity which must be controlled to particular levels specified in Official Monographs such as the European Pharmacopeia. For example, the European Pharmacopeia Monograph for Apomorphine Hydrochloride Hemihydrate details that the acceptance criterion for a detectable impurity such as Impurity C cannot be more than 0.1%. It is important to recognise, however, that the Official Monograph relates to apomorphine hydrochloride which is suitable for formulation and subsequent administration to a person. In this respect, the apomorphine hydrochloride ultimately prepared in a production campaign may have undergone several (or, indeed, many) purification treatments in order to reduce the level of Impurity C, as well as other impurities, to sufficiently acceptable low levels in order to conform to the required standard. The purification treatments therefore can typically result in extended processing times on plant and loss in product yield. In carrying out the process of the present invention, however, the formation of Impurity C can be minimised, thus reducing the requirement for further purification.

In one embodiment, the process provides apomorphine hydrochloride comprising Impurity C in an amount ≤ about 0.2 area % as determined by HPLC. In one embodiment, the process provides apomorphine hydrochloride comprising Impurity C in an amount ≤ about 0.19 area % as determined by HPLC. In one embodiment, the process provides apomorphine hydrochloride comprising Impurity C in an amount ≤ about 0.18 area % as determined by HPLC. In one embodiment, the process provides apomorphine hydrochloride comprising Impurity C in an amount ≤ about 0.17 area % as determined by HPLC. In one embodiment, the process provides apomorphine hydrochloride comprising Impurity C in an amount ≤ about 0.16 area % as determined by HPLC. In one embodiment, the process provides apomorphine hydrochloride comprising Impurity C in an amount ≤ about 0.15 area % as determined by HPLC. In one embodiment, the process provides apomorphine hydrochloride comprising Impurity C in an amount ≤ about 0.14 area % as determined by HPLC. In one embodiment, the process provides apomorphine hydrochloride comprising Impurity C in an amount ≤ about 0.13 area % as determined by HPLC. In one embodiment, the process provides apomorphine hydrochloride comprising Impurity C in an amount ≤ about 0.12 area % as determined by HPLC. In one embodiment, the process provides apomorphine hydrochloride comprising Impurity C in an amount ≤ about 0.11 area % as determined by HPLC. In one embodiment, the process provides apomorphine hydrochloride comprising Impurity C in an amount ≤ about 0.1 area % as determined by HPLC. A suitable HPLC method for determining the amount of Impurity C is, for example, the HPLC method detailed below.

In another aspect, the present invention provides a process for the preparation of a hydrochloric acid salt of compound of formula (2), the process comprising the steps of:

(a) heating calcium chloride and water to a temperature greater than 100 °C to form an aqueous calcium chloride solution;
(b) adding an aqueous hydrochloric acid solution of a compound of formula (1) to the aqueous calcium chloride solution;
(c) heating the reaction mixture of step (b) to a temperature greater than 100 °C to form the hydrochloric acid salt of the compound of formula (2); and
(d) treating the hydrochloric acid salt of compound (2) with a solvent mixture comprising an alcohol solvent and hydrochloric acid at a temperature greater than ambient temperature;

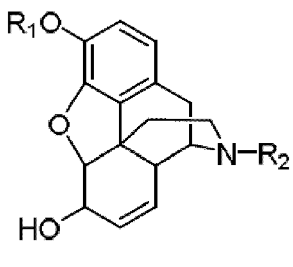

Compound of formula (1)

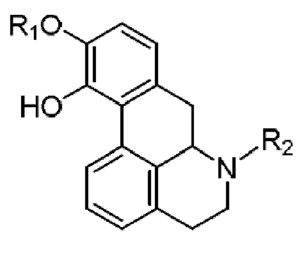

Compound of formula (2)

wherein:

$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and
$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1\text{-}20}$-alkyl-$C_{3\text{-}20}$-cycloalkyl, substituted -$C_{1\text{-}20}$-alkyl-$C_{3\text{-}20}$-cycloalkyl, unsubstituted allyl and substituted allyl.

All of the embodiments described above for steps (a), (b) and (c), as well as the treating step (described here as step (d)) likewise apply to this aspect of the invention.

Embodiments and/or optional features of the invention have been described above. Any aspect of the invention may be combined with any other aspect of the invention, unless the context demands otherwise. Any of the embodiments or optional features of any aspect may be combined, singly or in combination, with any aspect of the invention, unless the context demands otherwise.

The invention will now be described further by reference to the following examples, which are intended to illustrate but not limit, the scope of the invention.

## Examples

### HPLC Method

**Apomorphine Hydrochloride Hemihydrate European Pharmacopeia 9.0 Method**

| | |
|---|---|
| Column | : Waters Symmetry C18 5 microns 15 mm x 0.46 mm |
| Mobile Phase | : Prepare a solution as follows: |
| | : **A** 1.1 g/L octane sulfonic acid, sodium salt aqueous solution adjusted to pH 2.2 with 50% v/v phosphoric acid solution |
| | : **B** HPLC grade acetonitrile |
| Flow rate | : 1.5 ml / minute |
| Temperature | : 35 °C |
| Detector | : UV @ 280 nm |
| Injection Volume | : 10 microlitres |
| Run Time | : 37 minutes |

Gradient program:

| Time (min) | A % v/v | B % v/v |
|---|---|---|
| 0 | 85.0 | 15.0 |
| 2 | 85.0 | 15.0 |
| 32 | 68.0 | 32.0 |
| 37 | 68.0 | 32.0 |
| 45 | 85.0 | 15.0 |

**Sample Preparation**

**Sample Solution**

Approximately 25 mg of Apomorphine Hydrochloride was weighed into a 10 mL volumetric flask. The Apomorphine Hydrochloride was dissolved in and diluted to volume with 1% aqueous acetic acid.

**Reference Solution**

1.0 mL of the sample solution was diluted to 100 mL with 1% aqueous acetic acid, then further diluted 1.0 mL to 10 mL with 1% aqueous acetic acid (0.1%).

**Unspecified Impurities,** %

$$\text{Unspecified impurities (\%)} = \frac{\text{Impurity peak area in sample solution x 0.1}}{\text{Reference solution peak volume}}$$

**Calcium Chloride**

The calcium chloride used was calcium chloride dihydrate containing not more than 25% water.

**Example 1 (comparative)**

**Hydrochloric acid present in the calcium chloride solution**

Two 250 mL flange flasks were set up with condensers and temperature probes. Both were fitted with a nitrogen bubbler, and purged with nitrogen before starting the reaction.

Vessel A

1. Calcium chloride (80.0 g) was added to one of the 250 mL flasks.
2. Water (4 mL) was added to the flask.
3. Concentrated hydrochloric acid (8 mL) was added to the flask.
4. Water (4 mL) was added to the flask. The flask was purged with nitrogen.
5. The flask was heated to 145 °C, the stirrer was switched on when the temperature reached 70 °C. Significant quantities of hydrogen chloride gas were generated.

Vessel B

6. Water (5.3 mL) was added to the second 250 mL flask.
7. Concentrated hydrochloric acid (16.5 mL) was added to the same flask.
8. Water (7.1 mL) was added to the flask.
9. Stirring was started, and the flask was heated to 45 °C.
10. Morphine alkaloid (6.5 g) was added to the flask, and the flask purged with nitrogen.
11. The vessel's contents were heated to reflux to obtain a dark orange solution.

Reaction

12. The contents of Vessel B were added to Vessel A in small portions, at 145 °C. The slow addition of the morphine solution was to ensure that frothing/foaming was minimised as much as possible. It was noted that the temperature would increase to almost 150 °C when foaming, however, would then rapidly drop to 135 °C.

13. Water (1.8 mL) was added to Vessel A.
14. The reaction was stirred at 143 °C for 2 hours.

Work-up

15. Concentrated hydrochloric acid (7 mL) was dissolved in water (247 mL).
16. Dilute hydrochloric acid solution (23 mL, prepared in step 15) was added at 145 °C over 30 minutes. The temperature was maintained at > 100 °C, then allowed to heat back up to 145 °C.
17. The flask was cooled to 55 °C over 50 minutes to give a light brown suspension.
18. The contents of the flask were filtered using a 70 mm filter. There was a solid crust around the edge of the flask. This crust was scraped off and filtered with the mobile contents.
19. The filter cake was washed with dilute acid (4.5 mL, prepared in step 15), and sucked dry under vacuum overnight.
20. The solid isolated on the filter was sand-coloured. The filtrates were very thick and viscous (likely to be predominantly calcium chloride).
21. The isolated material was homogenised using a pestle and mortar to give a sand-coloured solid.
22. HPLC analysis was carried out on a sample of the solid. The HPLC assay yield was calculated to be 37% apomorphine. The levels of Impurity C were determined to be 0.66 area% (0.59 %w/w).

**Example 2 (according to the invention)**

**Removal of hydrochloric acid from the calcium chloride solution**

Two 250 mL flange flasks were set up with condensers and temperature probes. Both were fitted with a nitrogen bubbler, and purged with nitrogen before starting the reaction.

Vessel A

1. Calcium chloride (80.0 g) was added to one of the 250 mL flasks.
2. Water (16 mL) was added to the flask. The flask was purged with nitrogen.
3. The flask was heated to 145 °C, the stirrer was switched on when the temperature reached 70 °C. All solids were in solution at 145 °C. As concentrated hydrochloric acid was not used, no hydrogen chloride gas was produced on heating the flask's contents.

Vessel B

4. Water (5.3 mL) was added to the second 250 mL flask.
5. Concentrated hydrochloric acid (16.5 mL) was added to the same flask.
6. Water (7.1 mL) was added to the flask.
7. Stirring was started, and the flask was heated to 45 °C.
8. Morphine alkaloid (6.5 g) was added to the flask, and the flask purged with nitrogen.
9. The vessel's contents were heated to reflux to obtain a dark orange solution.

Reaction

10. The contents of Vessel B were added to Vessel A in small portions, at 145 °C. The slow addition of the morphine solution was to ensure that frothing/foaming was minimised as much as possible. It was noted that the temperature would increase to almost 150 °C when foaming, however, would then rapidly drop to 135 °C. Significantly reduced quantities of hydrogen chloride gas were produced on adding the contents of Vessel B to Vessel A.
11. Water (1.8 mL) was added to Vessel A.
12. The reaction was stirred at 143 °C for 2 hours.

Work-up

13. Concentrated hydrochloric acid (7 mL) was dissolved in water (247 mL).
14. Dilute hydrochloric acid solution (23 mL, prepared in step 13) was added at 145 °C over 30 minutes. The temperature was maintained at > 100 °C, then allowed to heat back up to 145 °C.
15. The flask was cooled to 55 °C over 50 minutes to give a light brown suspension.
16. The contents of the flask were filtered using a 70 mm filter. There was a solid crust around the edge of the flask. This crust was scraped off and filtered with the mobile contents.
17. The filter cake was washed with dilute acid (4.5 mL, prepared in step 13), and sucked dry under vacuum overnight.
18. The solid isolated on the filter was sand-coloured, with dark brown lumps of solid. The filtrates had set solid (likely to be predominantly calcium chloride).
19. The isolated material was homogenised using a pestle and mortar to give a sand-coloured solid.
20. HPLC analysis was carried out on a sample of the solid. The HPLC assay yield was calculated to be 41% apomorphine. The levels of Impurity C were determined to be 0.47 area% (0.46 %w/w).

The table below shows the impurity profiles of apomorphine hydrochloride (as determined by HPLC) when prepared by Example 1 (with hydrochloric acid in the calcium chloride solution) and Example 2 (without hydrochloric acid in the calcium chloride solution).

| **RRT** | **Component** | **Area %** | | **% w/w** | |
|---|---|---|---|---|---|
| | | **Example 2** | **Example 1** | **Example 2** | **Example 1** |
| | | **No HCl** | **HCl added** | **No HCl** | **HCl added** |
| 0.41 | | 0.08 | 0.07 | 0.08 | 0.06 |
| 0.45 | | 0.23 | 0.20 | 0.23 | 0.18 |
| 0.66 | | 0.11 | 0.13 | 0.11 | 0.12 |
| 1.00 | Apomorphine | 95.63 | 94.39 | | |
| 1.31 | | 0.02 | 0.20 | 0.02 | 0.18 |
| 1.32 | | 0.10 | | 0.10 | |
| 1.35 | | 0.01 | 0.08 | 0.01 | 0.07 |
| 1.4 | | 0.02 | 0.07 | 0.02 | 0.06 |
| 1.41 | | 0.08 | 0.09 | 0.08 | 0.08 |
| 1.44 | | 0.41 | 0.56 | 0.41 | 0.50 |
| 1.46 | | 0.33 | 0.28 | 0.33 | 0.25 |
| 1.48 | **Impurity C** | **0.47** | **0.66** | **0.46** | **0.59** |
| 1.51 | | 0.51 | 0.58 | 0.51 | 0.53 |
| 1.53 | | 0.30 | 0.42 | 0.30 | 0.38 |
| 1.55 | | 0.26 | 0.28 | 0.25 | 0.25 |
| 1.57 | | 0.37 | 0.35 | 0.36 | 0.32 |
| 1.65 | | 0.55 | 1.24 | 0.54 | 1.12 |
| 1.73 | | 0.08 | 0.13 | 0.08 | 0.12 |
| 1.77 | | 0.02 | 0.07 | 0.02 | 0.07 |
| 1.85 | | 0.04 | 0.10 | 0.04 | 0.09 |
| 1.94 | | 0.06 | 0.07 | 0.06 | 0.07 |
| | **Yield (%)** | 40.9 | 36.7 | | |

Example 2 shows that hydrochloric acid can be removed from the calcium chloride solution and replaced with water, without detriment to the impurity profile of apomorphine hydrochloride. The absence of hydrochloric acid from the calcium chloride solution is not detrimental to the yield of the process when compared to Example 1 where hydrochloric acid is present.

## Example 3

## Slurrying Investigations

### Example 3a (comparative)

1. Apomorphine hydrochloride (14.5 g) was added to a 250 mL flange flask.
2. Alcohol M (14 mL) and 37% hydrochloric acid (0.21 mL) were mixed together in a beaker. This solution was added to the flask containing the solid. The slurry was immediately mobile. Alcohol M is a mixture of 96% ethanol denatured with 4% methanol.
3. The slurry was cooled to 5 °C in an ice bath.
4. The slurry was left to stir for 30 minutes at 5 °C.
5. The solid was filtered at low temperature, and the filter cake was washed with Alcohol M (5 mL).
6. The solid was sucked dry under vacuum for 30 minutes. A light green/beige-coloured solid was obtained. The liquors were very dark brown.
7. The solid was transferred to a crystallisation dish, and left to dry in an oven at 40 °C overnight.
8. The solid was removed from the oven and weighed.
9. HPLC analysis was carried out on the isolated material.

### Example 3b (comparative)

1. Apomorphine hydrochloride (14.5 g) was added to a 250 mL flange flask.
2. Alcohol M (14 mL) and 37% hydrochloric acid (0.21 mL) were mixed together in a beaker. This solution was added to the flask containing the solid. The slurry was immediately mobile.
3. The slurry was heated to 30 °C, and left to stir for 30 minutes at this temperature.
4. The solid was filtered at 30 °C, and the filter cake was washed with Alcohol M (5 mL).
5. The solid was sucked dry under vacuum for 30 minutes.
6. A light green/beige-coloured solid was obtained. The liquors were very dark brown.
7. The solid was transferred to a crystallisation dish, and left to dry in an oven at 40°C overnight.
8. The solid was removed from the oven and weighed.
9. HPLC analysis was carried out on the isolated material.

### Example 3c (according to the invention)

1. Apomorphine hydrochloride (14.5 g) was added to a 250 mL flange flask.
2. Alcohol M (14 mL) and 37% hydrochloric acid (0.21 mL) were mixed together in a beaker. This solution was added to the flask containing the solid. The slurry was immediately mobile.
3. The slurry was heated to 60 °C. The temperature increased rapidly to 70 °C.
4. The slurry was allowed to cool to 41 °C, then heated back to 60 °C. Heating took approximately 10 minutes to reach 60 °C. The slurry was left for 15 minutes to stir at temperature.
5. The slurry was allowed to cool to 30 °C.
6. The solid was filtered at 30 °C, and the filter cake was washed with Alcohol M (5 mL).
7. The solid was sucked dry under vacuum for 30 minutes. A light beige-coloured solid (lighter than all previous experiments) was obtained. The liquors were very dark brown.
8. The solid was transferred to a crystallisation dish, and left to dry in an oven at 40°C overnight.
9. The solid was removed from the oven and weighed.
10. HPLC analysis was carried out on the isolated material.

**Example 3d (according to the invention)**

1. Apomorphine hydrochloride (14.5 g) was added to a 250 mL flange flask.
2. Alcohol M (14 mL) and 37% hydrochloric acid (0.21 mL) were mixed together in a beaker. This solution was added to the flask containing the solid. The slurry was immediately mobile.
3. The slurry was heated to 60 °C.
4. The slurry was left to stir at this temperature for 30 minutes.
5. The slurry was allowed to cool to 30 °C.
6. The solid was filtered at 30 °C, and the filter cake was washed with Alcohol M (5 mL).
7. The solid was sucked dry under vacuum for 30 minutes. A light beige-coloured solid (lighter than all previous experiments) was obtained. The liquors were very dark brown.
8. The solid was transferred to a crystallisation dish, and left to dry in an oven at 40°C overnight.
9. The solid was removed from the oven and weighed.
10. HPLC analysis was carried out on the isolated material.

**Example 3e (according to the invention)**

1. Apomorphine hydrochloride (14.5 g) was added to a 250 mL flange flask.
2. Alcohol M (14 mL) and 37% hydrochloric acid (0.21 mL) were mixed together in a beaker. This solution was added to the flask containing the solid. The slurry was immediately mobile.
3. The slurry was heated to 60 °C.
4. The slurry was left to stir at this temperature for 30 minutes.
5. The slurry was allowed to cool to 30 °C by removing the isomantle. The flask was cooled further using an ice bath to 5 °C.
6. The solid was filtered at this low temperature, and the filter cake was washed with Alcohol M (5 mL).
7. The solid was sucked dry under vacuum for 30 minutes. A light beige-coloured solid (lighter than all previous experiments) was obtained. The liquors were very dark brown.
8. The solid was transferred to a crystallisation dish, and left to dry in an oven at 40°C overnight.
9. The solid was removed from the oven and weighed.
10. HPLC analysis was carried out on the isolated material.

The table below shows the impurity profiles of the apomorphine hydrochloride before and after the slurrying investigations (all as determined by HPLC).

| **RRT** | **Component** | **Area%** | | | | | |
|---|---|---|---|---|---|---|---|
| | | **Apomorphine HCl** | **Example 3a** | **Example 3b** | **Example 3c** | **Example 3d** | **Example 3e** |
| | | **Input** | **5 °C** | **30 °C** | **60 °C** | **60 °C** | **60 °C** |
| 0.40 | | | 0.01 | | | | |
| 0.43 | | | 0.01 | 0.02 | | | |
| 0.66 | | | 0.04 | 0.03 | | | |
| 0.89 | | | 0.01 | | | | 0.01 |
| 1.00 | Apomorphine | 97.94 | 97.98 | 98.28 | 99.48 | 99.16 | 99.36 |
| 1.15 | | | 0.02 | | | | 0.02 |
| 1.31 | | 0.13 | 0.12 | 0.11 | 0.04 | 0.06 | 0.03 |
| 1.34 | | 0.06 | 0.05 | 0.05 | 0.02 | 0.02 | 0.01 |
| 1.39 | | | 0.02 | 0.02 | | | |
| 1.40 | | 0.09 | 0.02 | 0.02 | | 0.04 | 0.03 |

(continued)

| RRT | Component | Area% | | | | | |
|---|---|---|---|---|---|---|---|
| | | **Apomorphine HCl** | **Example 3a** | **Example 3b** | **Example 3c** | **Example 3d** | **Example 3e** |
| | | **Input** | **5 °C** | **30 °C** | **60 °C** | **60 °C** | **60 °C** |
| 1.42 | | 0.26 | 0.24 | 0.21 | 0.06 | 0.10 | 0.07 |
| 1.44 | | 0.04 | 0.04 | 0.04 | | 0.01 | 0.01 |
| 1.47 | Impurity C | **0.30** | **0.27** | **0.25** | **0.10** | **0.14** | **0.11** |
| 1.49 | | 0.20 | 0.22 | 0.19 | 0.06 | 0.09 | 0.07 |
| 1.51 | | 0.12 | 0.10 | 0.09 | 0.03 | 0.04 | 0.03 |
| 1.54 | | 0.03 | 0.03 | 0.03 | | 0.02 | 0.01 |
| 1.56 | | 0.13 | 0.12 | 0.10 | 0.03 | 0.05 | 0.03 |
| 1.65 | | 0.45 | 0.37 | 0.33 | 0.10 | 0.16 | 0.11 |
| 1.71 | | 0.12 | 0.15 | 0.14 | 0.06 | 0.06 | 0.05 |
| 1.76 | | 0.03 | 0.03 | 0.02 | | 0.02 | 0.01 |
| 1.83 | | 0.05 | 0.05 | 0.03 | | 0.03 | 0.02 |
| 1.87 | | | 0.02 | 0.02 | | | |
| 1.93 | | 0.05 | 0.04 | 0.04 | 0.01 | 0.02 | 0.02 |
| 2.04 | | | 0.01 | | | | |
| | Yield (%) | | 71 | 70 | 65 | 66 | 67 |
| | Assay (%) | | 94.1 | 96.1 | 100.6 | 97.9 | 99.1 |
| | Yield based on total API Content | | 66.8 | 67.3 | 65.0 | 64.6 | 66.4 |

Example 3a (slurrying temperature = 5 °C) showed a very small decrease in Impurity C content. A small improvement was observed when the slurry temperature was increased to 30 °C (Example 3b). The level of Impurity C was greatly decreased when higher temperatures were used for the slurrying (Example 3c-3e).

The calculated assays show that using a higher temperature for slurrying results in higher apomorphine content. The product of Example 3a was shown to contain 94.1% apomorphine, however, the product of Example 3d contained 97.9% apomorphine. These results again show the benefits of carrying out the acid Alcohol M slurrying at a higher temperature.

Additionally, the appearance of the isolated apomorphine hydrochloride obtained from Examples 3a-3e differed significantly. The apomorphine hydrochloride obtained from the higher temperature slurry is closer to white in colour, compared to the light beige colour produced with the lower temperature slurry.

## Example 4

### Larger scale slurrying experiment

1. Apomorphine hydrochloride (30 g) was added to a 250 mL flange flask.
2. Alcohol M (29 mL) and 37% hydrochloric acid (0.43 mL) were mixed together in a beaker. This solution was added to the flask containing the solid. The slurry was immediately mobile.
3. The slurry was heated to 60 °C.
4. The slurry was left to stir at this temperature for 2 hours.
5. The slurry was allowed to cool to 30 °C.
6. The solid was filtered at 30 °C, and the filter cake was washed with Alcohol M (21 mL).
7. The solid was sucked dry under vacuum for 30 minutes. A light grey solid was obtained containing some of the

"crust" material could be seen in the filter cake. The liquors were very dark brown.

8. The solid was transferred to a crystallisation dish, and left to dry in an oven at 40°C overnight.
9. The solid was removed from the oven and weighed.
10. HPLC analysis was carried out on the isolated material.

The table below shows the impurity profiles of the apomorphine hydrochloride before and after the slurrying experiment (both as determined by HPLC).

| RRT | Component | Area% | | %w/w | |
|---|---|---|---|---|---|
| | | Apomorphine hydrochloride before slurrying | Apomorphine hydrochloride after slurrying | Apomorphine hydrochloride before slurrying | Apomorphine hydrochloride after slurrying |
| 1.00 | Apomorphine | 96.97 | | | |
| 1.33 | | 0.08 | | 0.07 | |
| 1.41 | | 0.03 | | 0.03 | |
| 1.43 | | 0.14 | | 0.13 | |
| 1.45 | | 0.39 | 0.05 | 0.34 | 0.04 |
| 1.47 | | 0.17 | | 0.15 | |
| 1.49 | **Impurity C** | **0.45** | **0.08** | **0.40** | **0.06** |
| 1.52 | | 0.40 | 0.05 | 0.35 | 0.04 |
| 1.54 | | 0.35 | 0.03 | 0.31 | 0.02 |
| 1.56 | | 0.17 | | 0.15 | |
| 1.58 | | 0.20 | | 0.18 | |
| 1.76 | | 0.41 | 0.04 | 0.36 | 0.03 |
| 1.69 | | 0.12 | | 0.11 | |
| 1.75 | | 0.06 | | 0.05 | |
| 1.96 | | 0.05 | | 0.05 | |
| | Yield (%) | | 67 | | |
| | Assay (%) | | 101 | | |

## Clauses

The invention will now be described with reference to the following numbered clauses.

1. A process for the preparation of a hydrochloric acid salt of compound of formula (2), the process comprising the steps of:

(a) heating calcium chloride and water to a temperature greater than 100 °C to form an aqueous calcium chloride solution;
(b) adding an aqueous hydrochloric acid solution of a compound of formula (1) to the aqueous calcium chloride solution; and
(c) heating the reaction mixture of step (b) to a temperature greater than 100 °C to form the hydrochloric acid salt of the compound of formula (2);

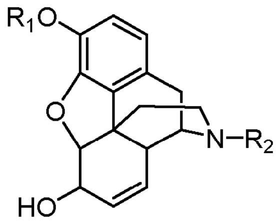 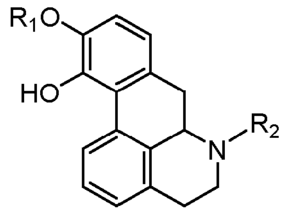

Compound of formula (1) Compound of formula (2)

wherein:

$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and

$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1\text{-}20}$-alkyl-$C_{3\text{-}20}$-cycloalkyl, substituted -$C_{1\text{-}20}$-alkyl-$C_{3\text{-}20}$-cycloalkyl, unsubstituted allyl and substituted allyl.

2. A process according to clause 1, wherein the calcium chloride and water is heated to one or more temperatures in the range of ≥ about 100 °C to about ≤ about 200 °C.

3. A process according to clause 2, wherein the calcium chloride and water is heated to about 145 °C.

4. A process according to any one of the preceding clauses, wherein the compound of formula (1) is:

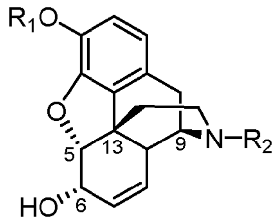

Compound of formula (1)

5. A process according to any one of the preceding clauses, wherein the compound of formula (2) is:

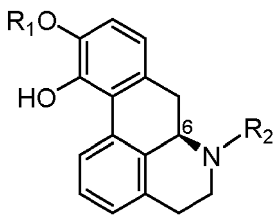

Compound of formula (2)

6. A process according to any one of the preceding clauses, wherein $R_1$ is H and $R_2$ is methyl.

7. A process according to any one of clauses 1 to 5, wherein $R_1$ and $R_2$ are methyl groups.

8. A process according to any one of the preceding clauses, wherein in step (c), the reaction mixture is heated to one or more temperatures in the range of ≥ about 100 °C to about ≤ about 200 °C.

9. A process according to clause 8, wherein the reaction mixture is heated to a temperature in the range of about 143 °C to about 145 °C.

10. A process for preparing a solid hydrochloric acid salt of compound (2), the process comprising the step of treating the hydrochloric acid salt of compound (2) with a solvent mixture comprising an alcohol solvent and hydrochloric acid at a temperature greater than ambient temperature,

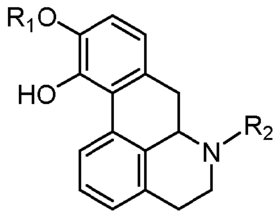

Compound of formula (2)

wherein:

$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and

$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1-20}$-alkyl-$C_{3-20}$-cycloalkyl, substituted -$C_{1-20}$-alkyl-$C_{3-20}$-cycloalkyl, unsubstituted allyl and substituted allyl.

11. A process according to clause 10, wherein the compound of formula (2) is:

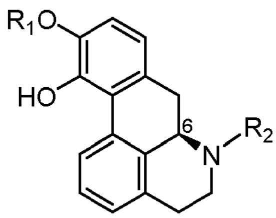

Compound of formula (2)

12. A process according to clause 10 or clause 11, wherein $R_1$ is H, and $R_2$ is methyl.

13. A process according to any one of clauses 1 to 5, wherein $R_1$ and $R_2$ are methyl.

14. A process according to any one of clauses 10 to 13, wherein the alcohol solvent is selected from the group consisting of methanol, ethanol or a mixture thereof.

15. A process according to any one of clauses 10 to 14, wherein the process is carried out at one or more temperatures in the range of ≥ about 40 °C to about ≤ about 70 °C.

16. A process according to clause 15, wherein the process is carried out at one or more temperatures in the range of ≥ about 55 °C to about ≤ 65 °C.

17. A process according to any one of clauses 10 to 16, further comprising the step of cooling the reaction mixture to ambient temperature or a temperature of less than ambient temperature.

18. A process according to clause 17, further comprising the step of recovering the hydrochloric acid salt of the compound of formula (2) by filtering, decanting or centrifuging.

19. A process for the preparation of a hydrochloric acid salt of compound of formula (2), the process comprising the steps of:

(a) heating calcium chloride and water to a temperature greater than 100 °C to form an aqueous calcium chloride solution;
(b) adding an aqueous hydrochloric acid solution of a compound of formula (1) to the aqueous calcium chloride solution; and
(c) heating the reaction mixture of step (b) to a temperature greater than 100 °C to form the hydrochloric acid salt of the compound of formula (2);
(d) treating the hydrochloric acid salt of compound (2) with a solvent mixture comprising an alcohol solvent and hydrochloric acid at a temperature greater than ambient temperature;

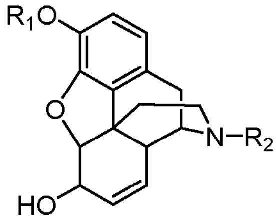

Compound of formula (1)

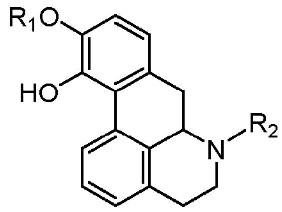

Compound of formula (2)

wherein:

$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and
$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1-20}$-alkyl-$C_{3-20}$-cycloalkyl, substituted -$C_{1-20}$-alkyl-$C_{3-20}$-cycloalkyl, unsubstituted allyl and substituted allyl.

## Claims

1. A process for preparing a solid hydrochloric acid salt of compound (2), the process comprising the step of treating the hydrochloric acid salt of compound (2) with a solvent mixture comprising an alcohol solvent and hydrochloric acid at a temperature greater than ambient temperature,

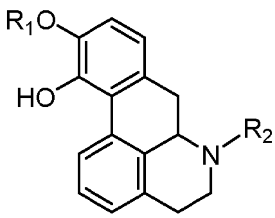

Compound of formula (2)

wherein:

$R_1$ is selected from the group consisting of -H, an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, and substituted cyclic $C_3$-$C_{20}$-alkyl; and
$R_2$ is selected from the group consisting of an unsubstituted straight-chain $C_1$-$C_{20}$-alkyl, substituted straight-chain $C_1$-$C_{20}$-alkyl, unsubstituted branched-chain $C_1$-$C_{20}$-alkyl, substituted branched-chain $C_1$-$C_{20}$-alkyl, unsubstituted cyclic $C_3$-$C_{20}$-alkyl, substituted cyclic $C_3$-$C_{20}$-alkyl, unsubstituted -$C_{1-20}$-alkyl-$C_{3-20}$-cycloalkyl, substituted -$C_{1-20}$-alkyl-$C_{3-20}$-cycloalkyl, unsubstituted allyl and substituted allyl.

2. A process according to claim 1, wherein the compound of formula (2) is:

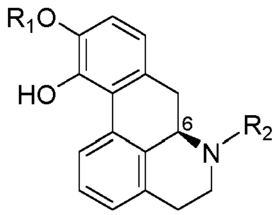

Compound of formula (2)

3. A process according to claim 1 or claim 2, wherein $R_1$ is H, and $R_2$ is methyl.

4. A process according to any one of claims 1 to 2, wherein $R_1$ and $R_2$ are methyl.

5. A process according to any one of claims 1 to 4, wherein the alcohol solvent is selected from the group consisting of methanol, ethanol or a mixture thereof.

6. A process according to any one of claims 1 to 5, wherein the process is carried out at one or more temperatures in the range of ≥ about 40 °C to about ≤ about 70 °C.

7. A process according to claim 6, wherein the process is carried out at one or more temperatures in the range of ≥ about 55 °C to about ≤ 65 °C.

8. A process according to any one of claims 1 to 7, further comprising the step of cooling the reaction mixture to ambient temperature or a temperature of less than ambient temperature.

9. A process according to claim 8, further comprising the step of recovering the hydrochloric acid salt of the compound of formula (2) by filtering, decanting or centrifuging.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SMALL et al.** *J. Org. Chem.*, 1940, vol. 5, 334